# EUROPEAN PATENT SPECIFICATION

(11) **EP 3 797 772 B1**
(45) Date of publication and mention of the grant of the patent: **18.10.2023**
(21) Application number: 19020504.7
(22) Date of filing: 03.09.2019
(51) Int. Cl.: A61K 31/4704, A61P 1/00, A61K 31/573

(54) **REBAMIPIDE FOR USE IN PREVENTION AND TREATMENT OF CROHN'S DISEASE**
REBAMIPID ZUR VERWENDUNG BEI DER PRÄVENTION UND BEHANDLUNG VON MORBUS CROHN
REBAMIPIDE DESTINÉ À ÊTRE UTILISÉ DANS LA PRÉVENTION ET LE TRAITEMENT DE LA MALADIE DE CROHN

(43) Date of publication of application: 31.03.2021
(73) Proprietor: SQUARE POWER LTD, London EC2A 3DQ (GB)
(72) Inventor:

(56) References cited:
- WO-A2-2005/072113
- KR-A- 20130 106 786
- KISHIMOTO S ET AL: "Therapeutic effect of rebamipide and 5-aminosalicylic acid on dextran sulfate sodium-induced rat colitis: Suppression of inflammatory cytokines", GASTROENTEROLOGY : OFFICIAL PUBLICATION OF THE AMERICAN GASTROENTEROLOGICAL ASSOCIATION, WILLIAMS & WILKINS, US, vol. 114, 15 April 1998 (1998-04-15), page A1010, XP027469012, ISSN: 0016-5085, DOI: 10.1016/S0016-5085(98)84111-5 [retrieved on 1998-04-15]
- DAVID LAHARIE ET AL: "Effect of Rebamipide on the Colonic Barrier in Interleukin-10-Deficient Mice", DIGESTIVE DISEASES AND SCIENCES, KLUWER ACADEMIC PUBLISHERS-PLENUM PUBLISHERS, NE, vol. 52, no. 1, 22 December 2006 (2006-12-22), pages 84-92, XP019464722, ISSN: 1573-2568
- T. MATYSIAK-BUDNIK ET AL: "Review article: rebamipide and the digestive epithelial barrier", ALIMENTARY PHARMACOLOGY & THERAPEUTICS., vol. 18, no. s1, July 2003 (2003-07), pages 55-62, XP055667986, GB ISSN: 0269-2813, DOI: 10.1046/j.1365-2036.18.s1.6.x
- ENTCHO KLENSKE ET AL: "Targeting mucosal healing in Crohn's disease: what the clinician needs to know", THERAPEUTIC ADVANCES IN GASTROENTEROLOGY, vol. 12, 14 June 2019 (2019-06-14), pages 1-11, XP055668477, UK ISSN: 1756-283X, DOI: 10.1177/1756284819856865

## Description

### Field of the Invention

The present invention relates to rebamipide for use in a method of prevention and/or treatment of Crohn's disease, in particular in a person suffering from increased intestinal permeability or in a person who is at risk of increased intestinal permeability.

### Background Art

Crohn's disease (CD) is an illness belonging to the group of inflammatory bowel diseases (IBD). It is a chronic multifactorial disorder, in which genetic, environmental, and microbial factors are involved. While the exact cause is unknown, it seems that the disease onset is triggered by environmental factors that perturb the mucosal barrier, alter the healthy balance of the gut microbiota, and abnormally stimulate gut immune responses. This leads to chronic inflammation of the intestine with typical symptoms such as diarrhea, weight loss, fatigue, rectal bleeding, and abdominal pain.

The disorder may occur at any age although it usually starts in the teens and twenties. It is estimated to affect about 3 in 1,000 people in Europe and North America with a similar frequency in males and females. It most often affects the end of the small intestine and the beginning of the colon, but it may also affect any part of the GI tract from the mouth to the anus. Skip lesions and patchy inflammation are a typical finding in CD.

At present there is no cure for Crohn's disease and current treatment aims at reducing the inflammation that triggers the symptoms. Over the years, several classes of medications have been developed for the treatment of CD. The choice of the medication depends upon the location of inflammation, severity of disease, complications, and the response of the patient to medical treatment. Most patients start with 5-aminosalicylates and antibiotics. Although mild disease can be treated with 5-aminosalicylates, many patients eventually require corticosteroids to control symptoms. They can help reduce inflammation and induce remission but their long-term use is associated with well known adverse effects. Moreover, about a half of patients is unable to discontinue corticosteroid therapy without disease exacerbation. Immunosuppressants, such as thiopurines and methotrexate, are frequently prescribed for patients who are resistant to or dependent on corticosteroids; however, these drugs have a slow onset of action and clinical remission rates of about 40%. Biological therapy using monoclonal antibodies against TNFα, such as infliximab (Remicade) and adalimumab (Humira), is used in patients unresponsive to conventional treatment with corticosteroids and immunosuppressants. Further option are antibodies targeting human integrin α₄β₇ resulting in gut-selective anti-inflammatory activity, such as vedolizumab (Entyvio).

Even though some of the treatments mentioned above may lead to a long-time relief from the symptoms of the disease, most patients with CD ultimately require surgery. Therefore, there is still a high unmet medical need for a treatment that would suppress intestinal inflammation and lead to complete remission of the disease symptoms.

Rebamipide, which is chemically 2-[(4-chlorobenzoyl)amino]-3-(2-oxo-1H-quinolin-4-yl)propanoic acid) is used for the treatment of acute and/or chronic gastritis and gastric ulcers. Its mechanism of action relates to mucosal defense, scavenging free radicals, and temporarily activating genes encoding cyclooxygenase-2.

LAHARIE, D. ET AL. Effect of rebamipide on the colonic barrier in interleukin-10-deficient mice. Dig. Dis. Sci. 2007, Vol. 52, No. 5, pages 84-92, discloses a treatment with rebamipide enema of interleukin-10-deficient mice infected with *Helicobacter hepaticus.* Since rectally administered rebamipide was found to reinforce the distal colonic barrier and have a slight Th1 immuno-stimulatory effect on mesenteric lymph node cells, it is suggested for the management of inflammatory bowel diseases associated with Th2-stimulation, such as ulcerative colitis.

MATYSIAK-BUDNIK T. ET AL. Review article: rebamipide and the digestive epithelial barrier. Aliment. Pharmacol. Ther. 2003, Vol. 18, Suppl. 1, pages 55-62, discloses that rebamipide shows protective properties with respect to the digestive epithelial barrier both *in vitro* and *in vivo,* and a capacity to diminish allergic sensitization and to promote oral tolerance to dietary antigens in mice. Since an excessive antigenic stimulation of the mucosal immune system is likely to play a role in the pathogenesis of food allergy and inflammatory bowel diseases, it is suggested that rebamipide may potentially be useful in the prevention and/or the treatment of these diseases.

### Summary of the Invention

It has been unexpectedly found that rebamipide, when administered to patients suffering from Crohn's disease, is able to suppress the disease symptoms and may even lead to complete remission of the disease. This is very surprising since it hasn't been reported that rebamipide, which is clinically used only for the treatment of gastric conditions, could be effective against Crohn's disease affecting mainly the intestine. Although rebamipide has been marketed since 1990, little is known about its behavior in the GI tract. The molecule is poorly absorbed in the GI tract, which indicates that following oral administration it may reach the intestinal mucosa in a concentration high enough to exert a therapeutic effect. Nevertheless, to the best of my knowledge, peroral rebamipide has never been reported as an effective treatment for Crohn's disease.

The mechanism of action is likely based on rebamipide's ability to induce mucine production in the intestine, to suppress inflammation and to restore the function of the tight junctions of epithelial cells, thus reducing the permeability of the intestinal wall and normalizing bowel function (Diao L et al. Rebamipide suppresses diclofenac-induced intestinal permeability via mitochondrial protection in mice. World J Gastroenterol. 2012;18(10):1059-1066). This leads to suppression of chronic inflammation and recovery of the intestine, preventing further tissue damage and reduction of the disease symptoms. Indeed, increased small intestinal permeability to various agents is a common observation in Crohn's disease patients (Katz KD et al. Intestinal permeability in patients with Crohn's disease and their healthy relatives. Gastroenterology. 1989; 97(4):927-931) and was reported to precede the onset of the disease in genetically predisposed individuals (Irvine EJ & Marshall JK. Increased intestinal permeability precedes the onset of Crohn's disease in a subject with familial risk. Gastroenterology. 2000;119(6),1740-1744).

The present invention thus provides rebamipide or a pharmaceutical composition thereof for use in a method of prevention and/or treatment of Crohn's disease, wherein the method is a combination therapy with at least one other drug effective against Crohn's disease that comprises the following steps:
- administering rebamipide as an add-on to a treatment with at least one other drug effective against Crohn's disease; and
- decreasing the dose of said other drug after symptom improvement.

"Rebamipide", as used herein, shall include all forms of this active ingredient, such as anhydrous form, hydrated or solvated form (e.g. hemihydrate form), crystalline forms; and pharmaceutically acceptable salts thereof.

"Prevention" or "preventive use" shall be understood herein as preventing or delaying the onset of the disease including its recurrence in patients with complete or partial remission after previous treatment. It is also meant to include maintenance of remission and/or non-worsening of the disease symptoms after discontinuation of previous treatment. Furthermore, it includes prevention of disease complications, such as bowel obstructions, ulcers and fistulas, anal fissure colon cancer, arthritis, uveitis, erythema nodosum, pyoderma gangrenosum, or apthous stomatitis.

"Treatment" shall be understood herein as a therapy that is able to abrogate, inhibit, slow or reverse the progression of the disease, and/or suppress chronic inflammation in the small intestine or elsewhere in the GI tract. It is also meant to cover amelioration or alleviation of clinical symptoms of the disease, such as abdominal pain, diarrhea, bloating, blood in stool, mouth sores, fatigue, reduced appetite and weight loss, malnutrition, skin inflammation, etc.

In the present invention , rebamipide is for use in a combination therapy with at least one other drug effective against Crohn's disease, wherein said other drug can be administered simultaneously, separately or sequentially to rebamipide. Particularly preferred drugs to be combined with rebamipide are corticosteroids.

The combination therapy comprises the steps of administering rebamipide as an add-on to a treatment with at least one other drug effective against Crohn's disease, especially 20 to 60 mg of prednisone or equivalent dose of another corticosteroid; and decreasing the dose of at least one other drug effective against Crohn's disease after symptom improvement, as can be defined by the decrease in CDAI by more than 100 points or by a decrease in fecal calprotectin by more than 150 µg/g. The at least one other drug may be completely discontinued after the patient reaches remission, as defined by endoscopy (mucosal healing) and/or CDAI lower than 150 points (clinical remission) and/or fecal calprotectin lower than 200 µg/g feces (inflammation suppression).

In one aspect of the invention, said one other drug effective against Crohn's disease is selected from 5-aminosalicylates, such as sulfasalazine or mesalazine; corticosteroids, such as prednisone, prednisolone, methylprednisolone, or budesonide; immunosuppressants, such as thiopurines (e.g. azathioprine, 6-mercaptopurine), methotrexate, cyclosporine or tacrolimus; antibiotics, such as metronidazole and ciprofloxacin; and antibodies (biologics), such as infliximab, adalimumab or vedolizumab. Corticosteroids are particularly preferred.

In one aspect of the invention, rebamipide is used in a person suffering from increased intestinal permeability or in a person who is at risk of increased intestinal permeability, e.g., due to family anamnesis or due to exposure to conditions or substances inducing increased intestinal permeability.

"Increased intestinal permeability" is used herein as a term designating little intestinal wall defects, including those caused by subclinical chronic inflammation (low grade inflammation) of the gut wall. These intestinal wall defects may be manifested by Crohn's disease but also by other medical conditions, such as chronic constipation or gastroparesis. Increased intestinal permeability may be diagnosed using specific tests, such as lactulose-mannitol test (LAMA test; e.g., Sequeira I.R. et al. (2014) PLoS One; 9(6):e99256), A-1-AT test, or zonulin test. Typically, increased intestinal permeability is permeability of the intestinal wall to particles having the size of more than 4 Angstroms in radius.

Substances inducing increased intestinal permeability include non-steroidal anti-inflammatory drugs (NSAIDs), such as acetylsalicylic acid, ibuprofen, naproxen, ketoprofen, fenoprofen, flurbiprofen, diclofenac, ketorolac, etodolac, indomethacin, tolmetin, piroxicam, meloxicam and selective COX-2 inhibitors such as celecoxib and etoricoxib; alcohol; nicotine; food additives; antibiotics; and chemotherapeutics. Thus, simultaneous or sequential coadministration of rebamipide with non-steroidal anti-inflammatory drugs, chemotherapeutics or antibiotics prevents intestinal wall damage and thus prevents or delays the onset of Crohn's disease associated with increased intestinal permeability. Prophylactic use of rebamipide may also be useful in persons abusing alcohol, nicotine or other drugs known to damage intestinal wall. The term "abuse" as used herein is meant to include any consumption, which is not necessary for medical reasons and leads to dependency and/or health impairments including low grade inflammation of the gut wall.

Conditions inducing increased intestinal permeability are related to stress, imbalanced diet, allergy, bacterial, viral or parasitic infections and various medical treatments. Such conditions in particular include stress-induced gastritis, alimentary intoxication, disbalance of cholic acids, gastric HCl and pepsin secretion, non-infectious diarrhea, radiation therapy, chemotherapy, infectious or post-infectious impairment of the GIT mucosa, dysmicrobia (e.g. induced by antibiotic treatment).

The person suffering from increased intestinal permeability typically suffers from at least one condition selected from low grade inflammation of the gut wall, chronic constipation or gastroparesis.

In the therapeutic indications as described in the present invention, rebamipide may preferably be used in pharmaceutical forms for oral administration such as tablets, capsules, dragees, granules, microgranules (sachets), orodispersible tablets or films, sublingual tablets, crushed tablets, oral solutions, oral suspensions, syrups, mouthwashes or rinses. Preferably, the oral pharmaceutical forms, such as tablets, capsules, dragees and granules, is a form with enteric release, such as enteric sustained release or enteric controlled release.

The pharmaceutical forms may contain at least one pharmaceutically acceptable excipient selected from fillers, binders, lubricants, glidants, disintegrants/swelling agents, solubilizers, enteric release agents, mucoadhesive components, sustained release agents, preservatives, coatings and colorants. Such excipients are known in the art of pharmaceutical formulation, and the skilled person is capable of selecting suitable excipients for the relevant pharmaceutical forms.

Suitable methods for preparing the pharmaceutical forms and compositions includes the processes of wet granulation or dry granulation of the active ingredient with the auxiliary substances and components, or direct homogenization of the active ingredient with the auxiliary substances and components.

Fillers may preferably be selected from saccharide alcohols (such as mannitol, sorbitol, xylitol), lactose, starch, pregelatinized starch, cellulose, sillicified cellulose, calcium hydrogen phosphate, calcium phosphate, sucrose and calcium sulphate. The fillers may preferably be present in the amount of 5 to 90 wt. %, relative to the total weight of the composition.

Binders may preferably be selected from starch, pregelatinized starch, povidone, copovidone, hydroxypropyl methylcellulose, hydroxypropyl cellulose, ethyl cellulose, cellulose. The binders may preferably be present in the amount of 1 to 20 wt. %, relative to the total weight of the composition.

Lubricants may preferably be selected from magnesium stearate, calcium stearate, stearic acid, polyethylene glycol and sodium stearyl fumarate. The lubricants may preferably be present in the amount up to 5 wt. %, relative to the total weight of the composition.

Glidants may preferably be selected from silica, talc and sodium lauryl sulphate. The glidants may preferably be present in the amount of 0.5 to 10 wt. %, relative to the total weight of the composition.

Swelling and/or disintegrating agents may preferably be selected from crospovidone, copovidone, povidone, croscarmellose, hydroxypropyl methylcellulose, starch, pregelatinized starch, low-substituted hydroxypropyl cellulose, sodium carboxymethyl starch. The swelling / disintegrating agents may preferably be present in the amount of 1 to 50 wt. %, relative to the total weight of the composition.

Solubilizers may preferably be selected from poloxamer, sodium lauryl sulphate, polysorbate, polyoxylated oleic glycerides, glycerol monostearate and cyclodextrins. The solubilizers may preferably be present in the amount up to 30 wt. %, relative to the total weight of the composition.

Enteric release agents may preferably be selected from hydroxypropyl methylcellulose phthalate, poly(methacrylic acid-co-methyl methacrylate), cellulose acetate phthalate, poly(vinyl acetate phthalate), esters of aleuritic acid. The enteric release agents may preferably be present in the amount of 2 to 40 wt. %, relative to the total weight of the composition.

Mucoadhesive components may preferably be selected from propylene glycol alginate, sodium alginate, calcium alginate, potassium alginate, hydroxypropyl methylcellulose, sodium carmellose, polyacrylic acid, polyethylene oxide, povidone and copovidone. The mucoadhesive components may preferably be present in the amount of 5 to 70 wt. %, relative to the total weight of the composition.

Sustained release agents may preferably be selected from cellulose and cellulose ethers such as hydroxypropyl cellulose, hydroxypropyl methylcellulose, carboxymethylcellulose, sodium carboxymethylcellulose, ethyl cellulose, methylcellulose, polyvinyl acetate, alginic acid, propylene glycol alginate, sodium alginate, calcium alginate, potassium alginate, polymethacrylates, guar gum, xanthan gum, carrageenan, castor oil, beeswax, carnauba wax, glycerol palmitostearate, glycerol monostearate, glycerol behenate, stearyl alcohol, polyacrylic acid. The sustained release agents may preferably be present in the amount of 5 to 70 wt. %, relative to the total weight of the composition.

The oral pharmaceutical composition may in some embodiments further contain a pharmaceutically acceptable component capable of forming carbon dioxide upon contact with gastric juices, such component may preferably be selected from carbonates and hydrogen carbonates of alkali metals and alkaline earth metals; and may preferably be present in an amount in the range from 1 to 50 wt. %, relative to the total weight of the composition.

A typical daily dose of rebamipide may range from 1 to 5000 mg for an average human (70 kg weight), more preferably from 50 to 2500 mg, even more preferably from 100 to 1000 mg, and most preferably from 300 to 600 mg or from 300 to 900 mg.

When immediate release formulation of rebamipide is administered, the daily dose is typically divided into several doses, which are administered separately. The daily dose may be divided into two to six separate doses taken twice daily or three times per day or four times per day or five times per day or six times per day. In a preferred embodiment the daily dose is divided into three separate doses administered three times per day, e.g. 100 mg dose administered three times per day. Alternatively, the whole daily dose can be taken at once, especially if it is in the form of a sustained release formulation, e.g. 300 mg dose administered once daily.

Hereinafter, the present invention will be described more specifically by the following working examples. However, the following working examples are provided only for illustrations and thus the present invention is not limited to it.

### Examples

Several patients suffering from Crohn's disease for at least 5 years without sufficient response to standard treatment were given rebamipide tablets three times a day. These patients had long history of treatment with various medicaments but were not able to reach remission after relapse of the disease using standard options. The severity of the disease was quantified by the CDAI, and the level of inflammation by fecal calprotectin.

Crohn's Disease Activity Index (CDAI) is a research tool used to quantify the symptoms of patients with Crohn's disease. It consists of eight factors, each summed after adjustment with a weighting factor. Remission of Crohn's disease is defined as CDAI below 150, whereas severe disease is defined as a value of greater than 450.

Calprotectin (FC) is a small calcium-binding protein that is used as a biomarker for inflammatory bowel diseases since its fecal concentration correlates well with the disease severity. Calprotectin values lower than 200 µg/g feces are considered the normal level. It was measured in stool samples using commercially available enzyme-linked immunosorbent assay (ELISA) kit (EK-CAL, Bühlmann).

### Example 1

A female patient, age 39, suffering from Crohn's ileocolitis, was diagnosed 16 years ago. Over the course of the disease she was given various medications including peroral, parenteral and topical corticosteroids, azathioprine, and TNFα inhibitors infiximab and adalimumab. After immunological response had developed against these antibodies, affecting skin and joints, the patient switched to vedolizumab (Entyvio), that was able to stabilize her for the next 22 months. During the relapse of the disease, the patient suffered with abdominal pain, increased stool frequency, joint and muscle pain, subfebrile fever, fatigue, and dry dermatitis with fecal calprotectin levels up to 1939 µg/g and CDAI of 358 points. Her treatment continued with methylprednisolon 24 mg/day without success. Concomitantly with the corticosteroid treatment she started being given rebamipide 100 mg three times daily resulting in fast remission of the disease as evidenced by alleviation of the disease symptoms (CDAI of 181 points) and decrease of calprotectin level to 228 µg/g after 6 weeks of the treatment. The corticosteroid dose was then reduced to half without any adverse effect. Corticosteroids were completely withdrawn after another 6 weeks when the calprotectin level lowered to 63 µg/g and CDAI to 73 points and the patient continued only rebamipide. No worsening in disease activity has been observed so far.

### Example 2

A male patient, age 27, was diagnosed with Crohn's ileocolitis 5 years ago and treated with mesalazine (Pentasa), budesonid, and metronidazole. He developed pancreatitis after three months on mesalazine. The medication was subsequently switched to azathioprine combined with prednison and later to methotrexate. However, the medication was not well tolerated and the patient was indicated for biological therapy. He was treated with infiximab (Remicade) and later with adalimumab (Humira). After the last relapse (calprotectin 2215 µg/g; CDAI of 479 points) he was given prednisone 40 mg/day but was not able to reach remission. He started taking rebamipide 200 mg t.i.d. as an add-on to prednisone. After 6 weeks (calprotectin 356 µg/g; CDAI of 225 points) the rebamipide dose was adjusted to 100 mg t.i.d. and prednisone dose to 30 mg/day. Prednisone dose was then lowered by 10 mg every 2 weeks and completely discontinued after another 6 weeks (calprotectin 110 µg/g; CDAI of 84 points). The patient continued only rebamipide 100 mg t.i.d. No worsening in disease activity has been observed so far.

### Example 3

A female patient, age 32, was diagnosed with Crohn's ileitis 12 years ago. Therapy with budesonide and mesalazine was able to maintain remission for about 10 years. She was thereafter hospitalized with abdominal pain and loose stool 3-4 times a day, with calprotectin level up to 1051 µg/g and CDAI of 377 points. She was prescribed azathioprine but it had to be discontinued after two weeks due to acute pancreatitis. Her treatment continued with prednisone 50 mg/day concomitantly with rebamipide 100 mg three times daily. The prednisone dose was adjusted to 25 mg after 14 days due to alleviation of the clinical symptoms and completely withdrawn after another 6 weeks with calprotectin level lowered to 122 µg/g and CDAI of 98 points and she continued only rebamipide. No worsening in disease activity has been observed so far.

The results obtained with the patient sample show that rebamipide is able to manage the disease symptoms, normalize calprotectin levels and induce remission even in those patients, who exhausted standard treatment options. The treatment started as an add-on to corticosteroid treatment due to ethical reasons but it is believed that it would be efficient even if prescribed as the only treatment. It is important to note that the corticosteroid treatment alone was not able to completely suppress the disease symptoms and neither patient had calprotectin lower than 200 µg/g feces, and CDAI lower than 150 points using only corticosteroids. Moreover, long-term therapy with rebamipide alone seems to be well tolerated and able to maintain remission.

## Claims

1. Rebamipide for use in a method of prevention and/or treatment of Crohn's disease, wherein the method is a combination therapy with at least one other drug effective against Crohn's disease that comprises the following steps:
- administering rebamipide as an add-on to a treatment with at least one other drug effective against Crohn's disease; and
- decreasing the dose of said other drug after symptom improvement.

2. Rebamipide for use according to claim 1, wherein said other drug is discontinued after the patient reaches remission.

3. Rebamipide for use according to any one of claims 1 or 2, wherein said other drug is selected from 5-aminosalicylates, preferably selected from sulfasalazine and mesalazine; corticosteroids, preferably selected from prednisone, prednisolone, methylprednisolone and budesonide; immunosuppressants, preferably selected from thiopurines, methotrexate, cyclosporine and tacrolimus; antibiotics, preferably selected from metronidazole and ciprofloxacin; and antibodies, preferably selected from infliximab, adalimumab or vedolizumab.

4. Rebamipide for use according to claim 1, wherein rebamipide is used in prevention and/or treatment of Crohn's disease in a person suffering from increased intestinal permeability or in a person who is at risk of increased intestinal permeability.

5. Rebamipide for use according to claim 4, wherein the person suffering from increased intestinal permeability is a person suffering from at least one condition selected from low grade inflammation of the gut wall, chronic constipation or gastroparesis.

6. Rebamipide for use according to claim 4, wherein the person at risk of increased intestinal permeability is a person suffering from stress, imbalanced diet, bacterial, viral or parasitic infection or a person exposed to at least one substance selected from non-steroidal anti-inflammatory drugs, alcohol, nicotine, food additives, chemotherapeutics and antibiotics.

7. Rebamipide for use according to claim 4, wherein the person at risk of increased intestinal permeability is a person suffering from or exposed to at least one condition selected from stress-induced gastritis, alimentary intoxication, disbalance of cholic acids, gastric HCl and pepsin secretion, non-infectious diarrhea, radiation therapy, chemotherapy, infectious or post-infectious impairment of the GIT mucosa, dysmicrobia.

8. Rebamipide for use according to any one of the preceding claims, wherein rebamipide is administered in an oral pharmaceutical form, preferably selected from tablets, capsules, dragees, granules, microgranules (sachets), orodispersible tablets or films, sublingual tablets, crushed tablets, oral solutions, oral suspensions, syrups, mouthwashes or rinses.

9. Rebamipide for use according to claim 8, wherein the oral pharmaceutical form is a tablet.

10. Rebamipide for use according to claim 8 or 9, wherein the oral pharmaceutical form is a form with enteric release, preferably enteric sustained release or enteric controlled release.

11. Rebamipide for use according to claim 8 or 9, wherein the pharmaceutical form contains rebamipide and at least one pharmaceutically acceptable excipient selected from fillers, binders, lubricants, glidants, disintegrants/swelling agents, solubilizers, enteric release agents, mucoadhesive components, sustained release agents, preservatives, coatings and colorants.

12. Rebamipide for use according to any one of the preceding claims, wherein rebamipide is administered in a daily dose of 1 to 5000 mg, more preferably from 50 to 2500 mg, even more preferably from 100 to 1000 mg, and most preferably from 300 to 600 mg.

## Patentansprüche

1. Rebamipide zur Verwendung in einer Methode zur Vorbeugung und/oder Behandlung von Morbus Crohn, wobei es sich bei der Methode um eine Kombinationstherapie mit mindestens einem anderen gegen Morbus Crohn wirksamen Arzneimittel handelt, die die folgenden Schritte umfasst:
- Verabreichung von Rebamipide als Ergänzung zu einer Behandlung mit mindestens einem anderen gegen Morbus Crohn wirksamen Arzneimittel; und
- Verringerung der Dosis des anderen Arzneimittels nach Besserung der Symptome.

2. Rebamipide zur Verwendung nach Anspruch 1, wobei das andere Arzneimittel abgesetzt wird, nachdem der Patient eine Remission erreicht hat.

3. Rebamipide zur Verwendung nach einem der Ansprüche 1 oder 2, wobei das andere Arzneimittel aus 5-Aminosalicylaten ausgewählt ist, vorzugsweise ausgewählt aus Sulfasalazin und Mesalazin; Kortikosteroide, vorzugsweise ausgewählt aus Prednison, Prednisolon, Methylprednisolon und Budesonid; Immunsuppressiva, vorzugsweise ausgewählt aus Thiopurinen, Methotrexat, Cyclosporin und Tacrolimus; Antibiotika, vorzugsweise ausgewählt aus Metronidazol und Ciprofloxacin; und Antikörper, vorzugsweise ausgewählt aus Infliximab, Adalimumab oder Vedolizumab.

4. Rebamipide zur Verwendung nach Anspruch 1, wobei Rebamipide zur Vorbeugung und/oder Behandlung von Morbus Crohn bei einer Person verwendet wird, die an einer erhöhten Darmpermeabilität leidet oder bei einer Person, bei der das Risiko einer erhöhten Darmpermeabilität besteht.

5. Rebamipide zur Verwendung nach Anspruch 4, wobei die Person, die an erhöhter Darmpermeabilität leidet, eine Person ist, die an mindestens einer Erkrankung leidet, ausgewählt aus einer leichten Entzündung der Darmwand, chronischer Verstopfung oder Gastroparese.

6. Rebamipide zur Verwendung nach Anspruch 4, wobei die Person, bei der das Risiko einer erhöhten Darmpermeabilität besteht, eine Person ist, die unter Stress, unausgewogener Ernährung, bakterieller, viraler oder parasitärer Infektion leidet oder eine Person ist, die mindestens einer Substanz, ausgewählt aus nichtsteroidalen entzündungshemmenden Mitteln, Drogen, Alkohol, Nikotin, Lebensmittelzusatzstoffen, Chemotherapeutika und Antibiotika ausgesetzt ist.

7. Rebamipide zur Verwendung nach Anspruch 4, wobei die Person, bei der das Risiko einer erhöhten Darmpermeabilität besteht, eine Person ist, die an mindestens einer Erkrankung leidet oder dieser ausgesetzt ist, ausgewählt aus stressbedingter Gastritis, Nahrungsmittelvergiftung, Ungleichgewicht von Cholsäuren, HCl- und Pepsin-Sekretion im Magen, nichtinfektiösem Durchfall, Strahlentherapie, Chemotherapie, infektiöser oder postinfektiöser Beeinträchtigung der Magen-Darm-Schleimhaut, Dysmikrobie.

8. Rebamipide zur Verwendung nach einem der vorhergehenden Ansprüche, wobei Rebamipide in einer oralen pharmazeutischen Form verabreicht wird, vorzugsweise ausgewählt aus Tabletten, Kapseln, Dragees, Granulaten, Mikrogranulaten (Beuteln), Schmelztabletten oder -filmen, Sublingualtabletten, zerstoßenen Tabletten, Lösungen zum Einnehmen, Suspensionen zum Einnehmen, Sirupe, Mundwässer oder Spülungen.

9. Rebamipide zur Verwendung nach Anspruch 8, wobei die orale pharmazeutische Form eine Tablette ist.

10. Rebamipide zur Verwendung nach Anspruch 8 oder 9, wobei die orale pharmazeutische Form eine Form mit enteraler Freisetzung, vorzugsweise enteraler verzögerter Freisetzung oder enteraler kontrollierter Freisetzung, ist.

11. Rebamipide zur Verwendung nach Anspruch 8 oder 9, wobei die pharmazeutische Form Rebamipide und mindestens einen pharmazeutisch verträglichen Hilfsstoff enthält, ausgewählt aus Füllstoffen, Bindemitteln, Gleitmitteln, Fließregulierungsmitteln, Sprengmitteln/Schwellmitteln, Lösungsvermittlern, magensaftresistenten Freisetzungsmitteln, mukoadhäsiven Komponenten und Wirkstoffen zur verzögerten Freisetzung , Konservierungsmitteln, Überzüge und Farbstoffe.

12. Rebamipide zur Verwendung nach einem der vorhergehenden Ansprüche, wobei Rebamipide in einer Tagesdosis von 1 bis 5000 mg, bevorzugter von 50 bis 2500 mg, noch bevorzugter von 100 bis 1000 mg und am meisten bevorzugt von 300 bis 600 mg verabreicht wird.

## Revendications

1. Rébamipide pour une utilisation dans une méthode de prévention et/ou de traitement de la maladie de Crohn où la méthode est une thérapie combinée avec au moins un autre médicament efficace contre la maladie de Crohn qui comprend les étapes suivantes :
- administrer du rébamipide en complément d'un traitement avec au moins un autre médicament efficace contre la maladie de Crohn ; et
- diminuer la dose dudit autre médicament après amélioration des symptômes.

2. Rébamipide pour une utilisation selon la revendication 1 où ledit autre médicament est arrêté après que le patient est en rémission.

3. Rébamipide pour une utilisation selon l'une quelconque des revendications 1 ou 2 où ledit autre médicament est choisi parmi les 5-aminosalicylates, de préférence choisis parmi la sulfasalazine et la mésalazine ; les corticostéroïdes, de préférence choisis parmi la prednisone, la prednisolone, la méthylprednisolone et le budésonide ; les immunosuppresseurs, choisis de préférence parmi les thiopurines, le méthotrexate, la cyclosporine et le tacrolimus ; les antibiotiques, de préférence choisis parmi le métronidazole et la ciprofloxacine ; et les anticorps, de préférence choisis parmi l'infliximab, l'adalimumab ou le vedolizumab.

4. Rébamipide pour une utilisation selon la revendication 1 où le rébamipide est utilisé dans la prévention et/ou le traitement de la maladie de Crohn chez une personne souffrant de perméabilité intestinale accrue ou chez une personne à risque de développer une perméabilité intestinale accrue.

5. Rébamipide pour une utilisation selon la revendication 4 où la personne souffrant de perméabilité intestinale accrue est une personne souffrant d'au moins un état choisi parmi une inflammation de bas grade de la paroi intestinale, une constipation chronique ou une gastroparésie.

6. Rébamipide pour une utilisation selon la revendication 4 où la personne à risque de développer une perméabilité intestinale accrue est une personne souffrant de stress, de déséquilibre alimentaire, d'infection bactérienne, virale ou parasitaire ou une personne exposée à au moins une substance choisie parmi les anti-inflammatoires non stéroïdiens, l'alcool, la nicotine, les additifs alimentaires, les chimiothérapies et les antibiotiques.

7. Rébamipide pour une utilisation selon la revendication 4 où la personne à risque de développer une perméabilité intestinale accrue est une personne souffrant de ou exposée à au moins une affection choisie parmi la gastrite induite par le stress, l'intoxication alimentaire, le déséquilibre des acides choliques, la sécrétion gastrique de HCl et de pepsine, la diarrhée non infectieuse, la radiothérapie, la chimiothérapie, l'altération infectieuse ou post-infectieuse de la muqueuse gastro-intestinale, la dysmicrobie.

8. Rébamipide pour une utilisation selon l'une quelconque des revendications précédentes où le rébamipide est administré sous une forme pharmaceutique orale, de préférence choisie parmi les comprimés, les gélules, les dragées, les granules, les microgranules (sachets), les comprimés ou films orodispersibles, les comprimés sublinguaux, les comprimés broyés, les solutions, suspensions buvables, les sirops, les bains de bouche ou les bans de rinçage.

9. Rébamipide pour une utilisation selon la revendication 8 où la forme pharmaceutique orale est un comprimé.

10. Rébamipide pour une utilisation selon la revendication 8 ou 9 où la forme pharmaceutique orale est une forme à libération entérique, de préférence à libération entérique prolongée ou à libération entérique contrôlée.

11. Rébamipide pour une utilisation selon la revendication 8 ou 9 où la forme pharmaceutique contient du rébamipide et au moins un excipient pharmaceutiquement acceptable choisi parmi les agents de remplissage, les liants, les lubrifiants, les agents de glissement, les agents désintégrants/agents gonflants, les solubilisants, les agents de libération entérique, les composants muco-adhésifs, les agents de libération prolongée, les conservateurs, les enrobages et les colorants.

12. Rébamipide pour une utilisation selon l'une quelconque des revendications précédentes où la dose quotidienne administrée de rébamipide est de 1 à 5000 mg, plus préférablement de 50 à 2500 mg, encore plus préférablement de 100 à 1000 mg, et le plus préférablement de 300 à 600 mg. mg.
